# EUROPEAN PATENT APPLICATION

(11) **EP 1 378 500 A1**
(43) Date of publication of application: **07.01.2004**
(21) Application number: 02014097.6
(22) Date of filing: 02.07.2002
(51) Int. Cl.: C07B 41/02, C07C 67/31, C07C 69/675, C07C 29/145, C07B 53/00

(54) **Diastereoselective hydrogenation of beta-hydroxy ketones**

(71) Applicant: Solvias AG, 4002 Basel (CH)
(72) Inventor: Studer, Martin, Dr., 4054 Basel (CH); Burkhardt, Stephan, 4460 Gelterkinden (CH)
(74) Representative: Dannappel, Hans-Jochen, Dr.

(57) **Abstract**

The invention relates to a novel process of diastereoselective hydrogenation of *1,3*-hydroxyketones of formula (I) 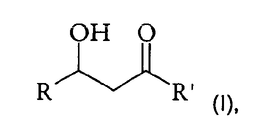 wherein R and R' are as defined in claim 1
which is carried out in the presence of a magnesium salt and a heterogeneous platinum catalyst in a solvent.

## Description

This invention relates to a novel process of diastereoselective hydrogenation of *1,3*-hydroxyketones.

Stereoseiective preparation of the 1,3-diol function has great utility in organic chemistry due to the occurrence of such a group in natural products. Besides enzymatic routes, there are also transition-metal-catalysed hydrogenation reactions described. For example, in Eur. J. Org. Chem. 1999, 1787-1793, the enantioselective homogeneous catalysis using rhodium- or ruthenium-bisphosphane complexes are described. However, the different proposed routes are not fully satisfactory.

It is therefore an object of the instant invention to provide for a hydrogenation process using a simple, commercially available heterogeneous catalyst which can be filtered off after the reaction.. It is a further object that the process has a good selectivity and that the hydrogenation proceeds fast. It is also an object to provide for a process not using expensive compounds. A further object is a process which does not necessarily require low temperature thus avoiding cooling equipment. An even further object is a process whereby the waste problems are minimised.

Surprisingly it has now been found that the addition of magnesium salts to a heterogeneous catalytic system based on platinum catalysts greatly improves the diastereoselective hydrogenation of 1,3-hydroxyketones.

The invention especially relates to a process, wherein a compound of formula (I) 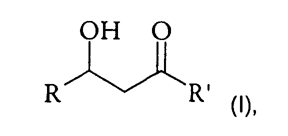 wherein R and R' are independently of each other a radical being compatible with the reaction conditions,
except compounds wherein
a) R is -CH₂CN and R' is -CH₂C(=O)OR_{b} and R_{b} is a H or a carboxy-protecting group;
b) R is -CH₂C(=O)NR*R** and R' is -CH₂C(=O)OR_{b} and R* and R** are independently of each other H or an amide-protecting group and R_{b} is H or a carboxy-protecting group;
c) R is -CH₂C(=O)OR_{b} and R' is -CH₂-CH₂-N₃ and R_{b} is H or a carboxy-protecting group;
d) R is -CH₂C(=O)OR_{b} and R' is -CH=CH-R_{c} and R_{b} is H or a carboxy-protecting group and R_{c} is H or halogen; and
e) R is -CH₂C(=O)OR_{b} and R' is -CH₂-CH₂-R_{d} and R_{b} is H or a carboxy-protecting group and R_{d} is halogen;

is reduced with hydrogen to the corresponding diol, which is predominantly in the form of the syn-diol, in the presence of a magnesium salt and a heterogeneous platinum catalyst in a solvent.

Unless otherwise indicated, halogen is preferably fluorine, chlorine, bromine or iodine.

Suitable compatible radicals (R or R') are for example alkyl, alkenyl, alkoxy, haloalkyl, hydroxyalkyl, alkoxyalkyl, haloalkoxy, cycloalkyl, cycloalkoxy, cycloalkyl-alkyl, cycloalkylalkoxy, aryl, aryloxy, aralkyl, aralkoxy, halogen, -OH, -OR₄, -OC(O)R₄, -NH-C(O)-R₄, -NR₄-C(O)-R₄, -CO₂R₄, -CO₂-NH₂, -CO₂-NHR₄, and -CO₂-NR₄R₅, wherein R₄ and R₅ are independently from each other C₁-C₄-alkyl, cyclohexyl, cyclohexylmethyl, phenyl or benzyl; to the extend possible said radicals may be branched or unbranched. Preferred radicals are alkyl (most preferred C₁-C₁₂-alkyl) and aryl (most preferred phenyl or naphthyl) which independently of each other may be unsubstituted or substituted. Suitable substituents are apparent from the given lists of compatible radicals and protecting groups. Preferred substituents are halogen, hydroxy and lower alkoxy.

The prefix "lower-" or "lower" indicates that the radical in question contains preferably up to 7 carbon atoms, especially up to 4 carbon atoms. Lower alkyl is therefore preferably C₁-C₇-alkyl, especially C₁-C₄-alkyl, and may be unbranched or branched one or more times, insofar as possible. Unsaturated radicals, such as alkenyl or alkynyl, have at least 2 carbon atoms, preferably from 2 to 7, especially from 3 to 7.

Carboxy-protecting groups are especially ester-forming, enzymatically and/or chemically removable protecting groups, preferably enzymatically and/or chemically removable protecting groups, such as heptyl, 2-N-(morpholino)ethyl, cholinyl, methoxyethoxyethyl or methoxyethyl; or those which are primarily chemically removable, e.g. alkyl, such as lower alkyl, especially methyl, ethyl, substituted lower alkyl (except for benzyl and substituted benzyl), such as substituted methyl, especially 9-fluorenylmethyl, methoxymethyl, methoxyethoxymethyl, methylthiomethyl, 2-(trimethylsilyl)ethoxymethyl, benzyloxymethyl, pivaloyloxymethyl, phenylacetoxymethyl, triisopropylsilylmethyl, 1-3-dithianyl-2-methyl, dicyclopropylmethyl, acetonyl, phenacyl, p-bromophenacyl, α-methylphenacyl, p-methoxyphenacyl, desyl, carbamidomethyl, p-azobenzenecarboxamidomethyl, N-phthalimidomethyl or 4-picolyl, 2-substituted ethyl, especially 2-iodo-, 2-bromo- or 2-chloro-ethyl, 2,2,2-trichloroethyl, 2-(trimethylsilyl)ethyl, 2-methylthioethyl, 2-(p-nitrophenylsulfenyl)ethyl, 2-(p-toluenesulfonyl)-ethyl, 2-(2'-pyridyl)ethyl, 2-(p-methoxyphenyl)ethyl, 2-(diphenylphosphino)ethyl, 1-methyl-1-phenylethyl, 2-(4-acetyl-2-nitrophenyl)ethyl or 2-cyanoethyl, tert-butyl, 3-methyl-3-pentyl, 2,4-dimentyl-3-pentyl or ω-chloro-lower alkyl, especially 4-chlorobutyl or 5-choropentyl, cyclopentyl, cyclohexyl, lower alkenyl, especially allyl, methallyl, 2-methylbut-3-en-2-yl, 3-methylbut-2-enyl or 3-buten-1-yl, substituted lower alkenyl, especially 4-(trimethylsilyl)-2-buten-1-yl, cinnamyl or α-methylcinnamyl, lower alkynyl, such as prop-2-ynyl, phenyl, substituted phenyl, especially 2,6-dialkylphenyl, such as 2,6-dimethylphenyl, 2,6-diisoproylphenyl, 2,6-di-tert-butyl-4-methylphenyl, 2,6-di-tert-butyl-4-methoxyphenyl, p-(methylthio)-phenyl or pentafluorophenyl, benzyl, substituted benzyl, especially triphenylmethyl, diphenylmethyl, bis(o-nitrophenyl)methyl, 9-anthrylmethyl, 2-(9,10-dioxo)anthrylmethyl, 5-dibenzosuberyl, 1-pyrenylmethyl, 2-(triflubromethyl)-6-chromonylmethyl, 2,4,6-trimethylbenzyl, p-bromobenzyl, o-nitrobenzyl, p-nitrobenzyl, p-methoxybenzyl, 2-6-dimethoxybenzyl, 4-(methylsulfinyl)benzyl, 4-sulfobenzyl, 4-azidomethoxybenzyl, 4-{N-[4,4-dimethyl,2,6-dioxocyclohexylidene)-3-methylbutyl]amino}benzyl, piperonyl or p-polymer-benzyl, tetrahydropyranol, tetrahydrofuranyl, or silyl radicals, such as tri-lower alkylsilyl, especially trimethylsilyl, triethylsilyl, tert-butyldiemethylsilyl, isopropyldimethylsilyl or di-tert-butylmethylsilyl, or phenyldi-lower alkylsilyl, such as phenyldimethylsilyl; alternatively a carboxy group can also be protected in the form of an oxazolyl, 2-alkyl-1,3-oxazolinyl, 4-alkyl-5-oxo-1,3-oxazolodinyl or 2,2-bistrifluoromethyl-4-alkyl-5-oxo-1,3-oxazolodinyl radical.

Amide-protecting groups are especially allyl, tert-butyl, N-methoxy, N-benzoyloxy, N-methylthio, triphenylmethylthio, tert-butyldimethylsilyl, triisopropylsilyl, 4-(methoxymethoxy)phenyl, 2-methoxy-1-naphthyl, 9-fluorenyl, tert-butoxycarbonyl, N-benzyloxycarbonyl, N-methoxy- or N-ethoxy-carbonyl, toluenesulfonyl, N-buten-1-yl, 2-methoxycarbonylvinyl, or especially alkyl, such as lower alkyl, or more especially substituted alkyl, especially benzyl, benzyl substituted by one ore more radicals selected from lower alkoxy, such as methoxy, lower alkanoyloxy, such as acetoxy, lower alkylsulfinyl, such as methylsulfinyl, dicyclopropylmethyl, methoxymethyl, methylthiomethyl and N-benzoyloxymethyl; or bis(trimethylsilyl)methyl, trichloroethoxymethyl, tert-butyldimethylsilyloxymethyl, pivaloyloxymethyl, cyanomethyl, benzyl, 4-methoxybenzyl, 2,4-dimethoxybenzyl, 3,4-dimethoxybenzyl, 2-acetoxy-4-methoxybenzyl, o-nitrobenzyl, bis(4-methoxyphenyl)phenylmethyl, bis(4-methylsulfinylphenyl)methyl, pyrrolidinomethyl, diethoxymethyl, 1-methoxy-2,2-dimethylpropyl or 2-(4-methylsulfonyl)ethyl.

It is a characteristic of protecting groups that they are simple to remove (that is to say without undesirable secondary reactions taking place), for example by solvolysis, reduction, photolysis or alternatively under conditions analogous to physiological conditions, for example enzymatically.

The instant hydrogenation process yields predominantly syn-diols. Depending on the substrate and the process conditions the syn-diol exeeds, for example, 60%, preferably 70% and most preferred 90%, of the total diol produced.

Suitable solvents are alcohols, especially lower alkanoles such as methanol, ethanol, propanol or butanol, or ethylenglykol, diethylenglykol, ethylenglykolmonomethyl- or monoethylether, diethylenglykolmonomethyl- or monoethyletheror ketones such as acetone or methylisobutylketone. The solvent may also be a mixture of solvents or a mixture of a solvent or solvents with water.

Platinum catalysts are known per se, are well described in the literature and are commercially available. It is possible to use platinum in the form of the pure metal, for example as a powder, or, what is preferred, in the form of finely distributed metal on a support. A suitable support material is for example carbon, metal oxides like SiO₂, TiO₂, Al₂O₃, metal salts, and natural or synthetic silicates. The catalyst may also be in the form of colloidal platinum. The amount of platinum metal is for example 1 to 10 % by weight, preferably 3 to 8 % by weight, relative to the support.

The hydrogenation is carried out for example with a hydrogen pressure of up to 200 bar, preferably with a hydrogen pressure of 1 to 200 bar and most preferred with a hydrogen pressure of 5 to 20 bar.

Suitable magnesium salts are the customary salts, for example magnesium acetate, magnesium chloride, magnesium bromide, magnesium ascorbate, magnesium gluconate, magnesium stearate, magnesium nitrate, magnesium sulfate and magnesium citrate whereby magnesium acetate is particularly preferred.

A suitable ratio by weight of the magnesium salt to the heterogeneous platinum catalyst in the instant process is from 10:1 to 1:10, preferably from 5:1 to 1:5, and most preferred from 5:1 to 1:2, whereby for above calculation purpose the magnesium salt is in the form of magnesium acetate and the heterogeneous catalyst is in the form of a carbon support with 5% by weight of platinum.

Compounds of formula (I) are preferred wherein R or R' is -C(=O)OR_{b} and R_{b} is H or a carboxy-protecting group.

Compounds of formula (I) are preferred wherein R is -C(=O)OR_{b};
R' is substituted or unsubstituted alkyl or substituted or unsubstituted aryl and
R_{b} is H or a carboxy-protecting group

### Examples

### H1:

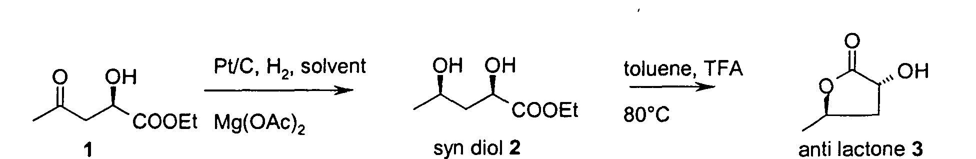

100 mg 5% Pt/C (Engelhard 4709) and 100 mg Mg(OAc)₂ are placed in a 2.5 ml vial equipped with a small magnetic stirrer bar. After the addition of 1 ml EtOH, 100 micro liter **1** are added. The vial is placed in a 50 ml autoclave, the autoclave is sealed, purged with argon (3 times) and with hydrogen (3 times). Then the autoclave is pressurized to 20 bar and the magnetic stirring is started. The reaction is run for 19 h at room temperature (20 to 25°C). Then the pressure is released, and the autoclave is purged with argon. The product is filtered and evaporated to dryness giving a mixture of **2** and **3** in quantitative yield This mixture is dissolved in 2 ml toluene. After the addition of 20 micro liters of trifluoro acetic acid, the mixture is stirred at 80°C over night. The reaction mixture is again evaporated to dryness and analyzed by 13-C NMR. Interpretation according to V. Blandin, J.-F. Carpentier, and A. Mortreux, Eur. J. Org. Chem 1999 (1787). Results: The analysis of the 13-C NMR shows 100% conversion and a 30 / 70 ratio of the anti / syn lactone 3, resulting from a 70 / 30 syn / anti diol mixture.

### H2:

The reaction is carried out as described in H1, without the addition of the Mg(OAc)₂ salt.

Results: The analysis of the 13-C NR shows 100% conversion and a 43 / 57 ratio of the anti / syn lactone **3**, resulting from a 57 / 43 syn / anti diol mixture.

### H3-H6:

Table 1 list conversions of various experiments to show the accelerating effect of Mg(OAc)₂. The conditions are the same as described in H1 and H2 with the changes noted in table 1.

**Table 1:**

| Cat. (mg) | Additive | Add (mg) | solvent | time, min | conv. % 1 GC | Example |
|---|---|---|---|---|---|---|
| 20 | - | - | MeOH | 120 | 4 | H3 |
| 20 | Mg(OAc)₂ | 5 | MeOH | 120 | 13 | H4 |
| 100 | - | - | MeOH | 1260 | 36 | H5 |
| 100 | Mg(OAc)₂ | 100 | MeOH' | 1260 | 97 | H6 |

## Claims

1. A process, wherein a compound of formula (I) 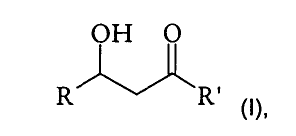 wherein R and R' are independently of each other a radical being compatible with the reaction conditions,
except compounds wherein
a) R is -CH₂CN and R' is -CH₂C(=O)OR_{b} and R_{b} is a H or a carboxy-protecting group;
b) R is -CH₂C(=O)NR*R** and R' is -CH₂C(=O)OR_{b} and R* and R** are independently of each other H or an amide-protecting group and R_{b} is H or a carboxy-protecting group;
c) R is -CH₂C(=O)OR_{b} and R' is -CH₂-CH₂-N₃ and R_{b} is H or a carboxy-protecting group;
d) R is -CH₂C(=O)OR_{b} and R' is -CH=CH-R_{c} and R_{b} is H or a carboxy-protecting group and R_{c} is H or halogen; and
e) R is -CH₂C(=O)OR_{b} and R' is -CH₂-CH₂-R_{d} and R_{b} is H or a carboxy-protecting group and R_{d} is halogen;
is reduced with hydrogen to the corresponding diol, which is predominantly in the form of the syn-diol, in the presence of a magnesium salt and a heterogeneous platinum catalyst in a solvent.

2. A process according to claim 1, wherein the magnesium salt is magnesium acetate.

3. A process according to claim 1 wherein the support material for the heterogeneous platinum catalyst is selected from carbon or Al₂O₃.

4. A process according to claim 1 wherein the solvent is an alcohol or a mixture of an alcohol with water.

5. A process according to claim 1 wherein the hydrogen pressure is up to 200 bar, especially 5 to 20 bar.

6. A process according to claim 1 wherein the reaction is carried out at a temperature of between 0 and 80°C, especially at 20 to 25°C.

7. A process according to claim 1 wherein
R or R' is -C(=O)OR_{b} and R_{b} is H or a carboxy-protecting group.

8. A process according to claim 1 wherein
R is -C(=O)OR_{b};
R' is substituted or unsubstituted alkyl or substituted or unsubstituted aryl and
R_{b} is H or a carboxy-protecting group
